# FASCICULE DE BREVET EUROPEEN

(11) **EP 3 233 296 B1**
(45) Date de publication et mention de la délivrance du brevet: **11.09.2019**
(21) Numéro de dépôt: 15828346.5
(22) Date de dépôt: 16.12.2015
(51) Int. Cl.: B05B 11/02, A61M 5/32, B21G 1/08

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 16.12.2014 FR 1462489
(43) Date de publication de la demande: 25.10.2017
(73) Titulaire: Aptar France SAS, 27110 Le Neubourg (FR)
(72) Inventeur: PETIT, Ludovic, 27110 Vitot (FR)
(74) Mandataire: CAPRI
(86) Numéro de dépôt international: PCT/FR2015/053543
(87) Numéro de publication internationale: WO 2016/097603

(56) Documents cités:
- EP-A1- 0 495 214
- EP-A1- 0 546 607
- WO-A1-90/01349
- WO-A1-2004/064903
- DE-A1- 4 412 041
- DE-A1- 10 224 101
- US-A1- 2003 153 879

## Description

La présente invention concerne un dispositif de distribution de produit fluide, et plus particulièrement un dispositif du type unidose ou bidose, adapté à distribuer une ou deux doses de produit fluide en un seul actionnement.

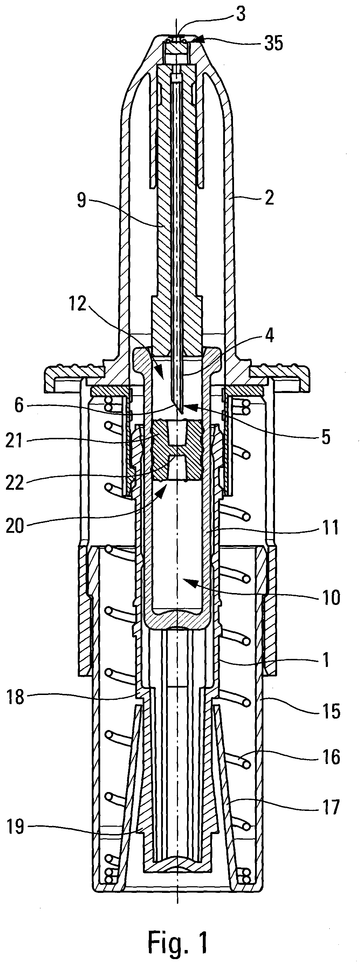

Les dispositifs du type unidoses ou bidoses sont bien connus. Comme visible sur la figure 1, ils comportent généralement un corps 1 recevant un réservoir 10 contenant une ou deux doses de produit fluide, et une tête de distribution 2 pourvue d'un orifice de distribution 3, et qui est déplaçable axialement par rapport audit corps 1 lors de l'actionnement. Le réservoir 10 est généralement formé par un tube creux borgne 11 dont l'ouverture axiale proximale 12 (par rapport à l'orifice de distribution 3) est fermée de manière étanche par un bouchon 20. La tête de distribution 2 comporte généralement une canule ou aiguille 4, de forme généralement cylindrique, reliée d'un côté audit orifice de distribution 3, et pourvue de l'autre côté d'une pointe de perçage 5 adaptée à percer ledit bouchon 20 lors de l'actionnement, le bouchon 20 se déplaçant alors dans ledit réservoir 10 pour expulser la ou les doses de produit fluide à travers ladite canule 4 vers ledit orifice de distribution 3. Les documents DE4412041 et EP0546607 décrivent des dispositifs de ce type.

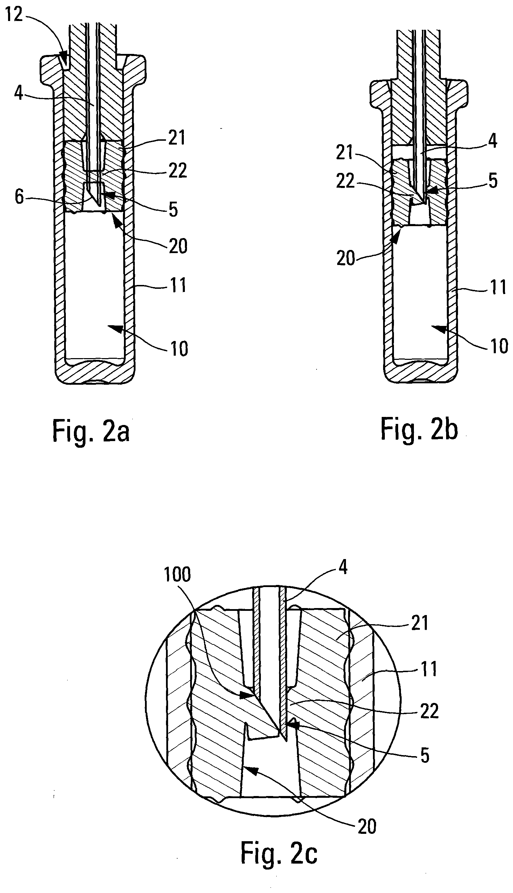

Un inconvénient avec ces dispositifs de l'art antérieur concerne les risques de fuites lors de l'actionnement, ce qui altère la dose distribuée et la reproductibilité du dosage entre différents dispositifs du même type. En particulier, comme visible sur la figure 2a, l'ouverture 6 dans la pointe de perçage 5 de la canule 4 est généralement formée par un biseau de l'extrémité axiale distale (par rapport à l'orifice de distribution 3) de la canule, formant ainsi un bord périphérique 7, qui entoure ladite ouverture 6, dont la surface est plane. Pour faciliter le perçage du bouchon 20, l'angle dudit biseau est relativement aigu, de sorte que l'étendue axiale de ladite ouverture 6, c'est-à-dire la distance axiale entre l'extrémité axiale distale et l'extrémité axiale proximale dudit bord périphérique 7, peut être supérieure à l'épaisseur de la paroi 22 du bouchon 20 qui est percée par ladite canule 4 au début de l'actionnement. Ceci peut donc occasionner des fuites au moment du perçage du bouchon 20, avec du fluide qui peut s'écouler hors du réservoir 10 sans passer dans ladite canule 4. De plus, comme visible sur les figures 2b et 2c, avec une canule standard comme celle de la figure 2a, il y a un risque de détérioration de la zone charnière 100 proche de l'extrémité axiale proximale dudit bord périphérique de l'ouverture, générant des fuites potentielles.

Les documents DE10224101et US2009163877 décrivent d'autres dispositifs de l'art antérieur.

La présente invention a pour but de fournir un dispositif de distribution de produit fluide, notamment du type unidose ou bidose, qui ne reproduit pas les inconvénients susmentionnés.

La présente invention a aussi pour but de fournir un dispositif de distribution de produit fluide, notamment du type unidose ou bidose, qui améliore la précision du dosage et sa reproductibilité pour une pluralité de dispositifs du même type, en limitant voire éliminant les risques de fuites lors de l'actionnement.

La présente invention a également pour but de fournir un dispositif de distribution de produit fluide, notamment du type unidose ou bidose, qui est simple et peu coûteux à fabriquer et à assembler.

La présente a donc pour objet un dispositif de distribution de produit fluide comportant un corps et une tête de distribution pourvue d'un orifice de distribution et déplaçable axialement par rapport audit corps lors de l'actionnement, ledit corps recevant un réservoir contenant du produit fluide, ledit réservoir comportant un tube creux ayant une ouverture axiale proximale fermée par un bouchon adapté à coulisser de manière étanche dans ledit tube lors de l'actionnement, ladite tête de distribution comportant une canule creuse reliée d'un côté audit orifice de distribution et pourvue de l'autre côté d'une pointe de perçage pourvue d'une ouverture pour percer ledit bouchon, ladite ouverture définissant un bord périphérique s'étendant autour de ladite ouverture et comportant une extrémité axiale distale et une extrémité axiale proximale, ladite extrémité axiale distale dudit bord périphérique formant l'extrémité axiale distale de la pointe de perçage, ladite extrémité axiale distale de la pointe de perçage étant disposée sur l'axe central longitudinal de ladite canule, caractérisé en ce que ladite ouverture comporte une partie distale de largeur réduite par rapport à la largeur de ladite canule, ledit bord périphérique comportant un rétrécissement radial pour définir ladite partie distale de largeur réduite de ladite ouverture, l'extrémité axiale distale de ladite pointe de perçage étant de forme arrondie dans la direction axiale.

Avantageusement, ledit bord périphérique est incurvé axialement.

Avantageusement, ladite pointe de perçage est formée par matriçage de ladite canule.

Avantageusement, ladite pointe de perçage a une forme externe arrondie dans la direction radiale.

Avantageusement, ledit réservoir est formé par un tube creux borgne.

Avantageusement, ledit réservoir contient une dose unique de produit fluide distribuée lors d'un seul actionnement du dispositif.

Avantageusement, ledit réservoir contient deux doses de produit fluide distribuées lors de deux actionnements successifs du dispositif.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique d'un dispositif de distribution de type bidose auquel peut s'appliquer la présente invention ;
- la figure 2a est une vue de détail en section transversale d'un dispositif standard de l'art antérieur;
- les figures 2b et 2c sont des vues de détails du dispositif de la figure 2a, en cours de perçage du bouchon,
- la figure 3 est une vue similaire à celle de la figure 2a, illustrant un mode de réalisation avantageux de la présente invention;
- les figures 4 à 6 sont des vues de détail de la canule de la figure 3; et
- la figure 7 est une vue schématique d'un ensemble de matriçage avantageux pour réaliser le mode de réalisation des figures 3 à 6.

La présente invention concerne plus particulièrement un dispositif du type de celui divulgué dans les documents DE4412041 et EP0546607.

Il est toutefois entendu que la présente invention ne se limite pas à ce type de dispositif, mais est au contraire applicable à tous les types de dispositifs de distribution de produit fluide, du type unidose ou bidose ou du type multidoses, comportant un réservoir obturé par un bouchon, ledit bouchon devant être percé avant ou au début de l'actionnement.

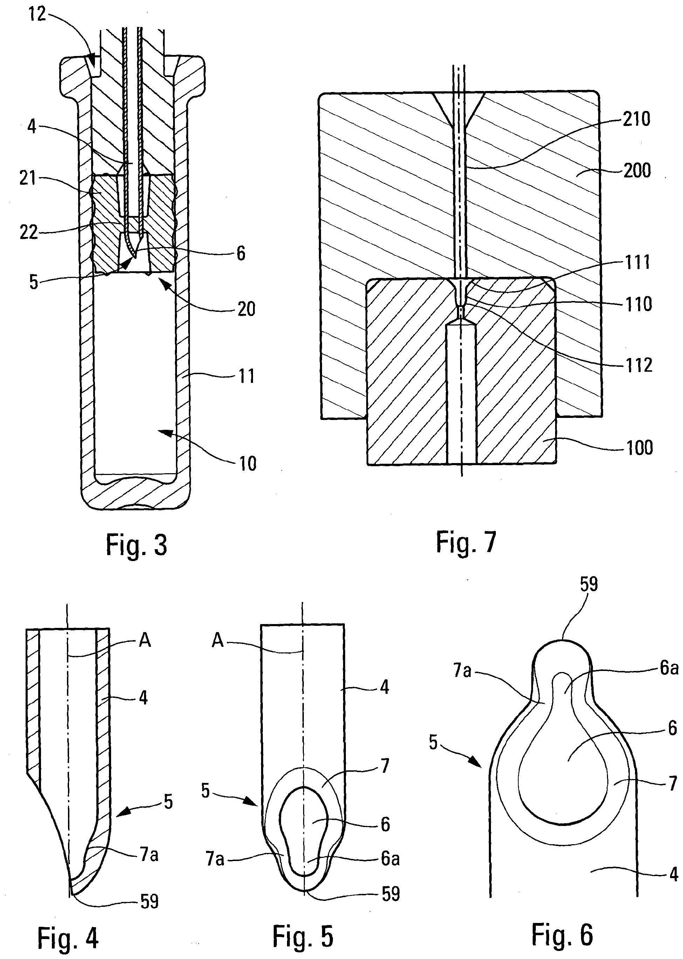

Dans la description, les termes "axial" et "radial" se réfèrent en particulier à l'axe central longitudinal A de la canule 4, visible sur les figures 4 et 5. Les termes "proximal" et "distal" se réfèrent à l'orifice de distribution 3 formé dans la tête de distribution 2.

L'invention s'applique plus particulièrement à des dispositifs du type unidose ou bidose tel que celui représenté sur la figure 1. Comme décrit précédemment, un tel dispositif comporte un corps 1 qui reçoit un réservoir 10 contenant une ou deux doses de produit fluide, et une tête de distribution 2, pourvue d'un orifice de distribution 3, est déplaçable axialement par rapport audit corps 1 lors de l'actionnement.

Le réservoir 10 contient une ou deux dose(s) de produit fluide. Avantageusement, ledit réservoir 10 est formé par un tube creux borgne 11, par exemple en verre, ayant une ouverture axiale proximale 12 fermée par un bouchon 20, par exemple en élastomère, adapté à coulisser de manière étanche dans ledit tube 11 lors de l'actionnement. Ledit bouchon 20 comporte une partie axiale tubulaire 21 coulissant de manière étanche dans le tube 11, et une paroi transversale 22 destinée à être percée lors de l'actionnement.

La tête de distribution 2 comporte généralement une canule ou aiguille creuse 4, de forme généralement cylindrique, reliée d'un côté audit orifice de distribution 3, et pourvue de l'autre côté d'une pointe de perçage 5 adaptée à percer ledit bouchon 20 lors de l'actionnement, le bouchon 20 se déplaçant alors dans ledit réservoir 10 pour expulser la ou les doses de produit fluide à travers ladite canule 4 vers ledit orifice de distribution 3. La canule 4 peut être insérée dans un support de canule 9, qui lui-même peut être fixé dans ladite tête de distribution 2. Avantageusement, un profil de pulvérisation 35 peut être formé directement en amont de l'orifice de distribution 3, par exemple entre le fond de ladite tête de distribution 2 et l'extrémité axiale proximale dudit support de canule 9.

La pointe de perçage 5 est pourvue d'une ouverture 6 pour percer ledit bouchon 20, ladite ouverture 6 définissant un bord périphérique 7 s'étendant autour de ladite ouverture 6 et comportant une extrémité axiale distale et une extrémité axiale proximale.

Un organe d'actionnement 15, connecté audit corps 1, peut être prévu pour réaliser l'actionnement. Dans l'exemple représenté sur la figure 1, l'organe d'actionnement 15 est sollicité vers sa position de repos par un ressort 16, et est déplaçable manuellement, contre la force du ressort 16, pour déplacer le corps 1 avec le réservoir 10 par rapport à la tête de distribution 2. L'exemple de la figure 1 étant un bidose, l'organe d'actionnement comporte des moyens de fractionnement de dose, formés dans cet exemple sous la forme d'une ou plusieurs pattes flexibles 17 coopérant avec des épaulements radiaux respectifs 18, 19 formés sur ledit corps 1. Ainsi, pour distribuer la première dose, la patte flexible 17 va coopérer avec l'épaulement 18, puis le ressort 16 va ramener l'organe d'actionnement 15 en position de repos, dans laquelle ladite patte flexible coopérera avec le second épaulement 19. D'autres moyens de fractionnement de dose sont envisageables.

Les figures 3 à 6 illustrent un mode de réalisation avantageux de la présente invention.

Selon ce mode de réalisation, ladite extrémité axiale distale dudit bord périphérique 7 forme l'extrémité axiale distale 59 de la pointe de perçage 5, et ladite extrémité axiale distale 59 de la pointe de perçage 5 est disposée sur l'axe central longitudinal A de ladite canule 4.

Ladite ouverture 6 comporte une partie distale 6a de largeur réduite par rapport à la largeur de ladite canule 4, définie par un rétrécissement radial 7a dudit bord périphérique 7.

L'invention permet donc de générer un profil de coupe qui est bien plus petit, en particulier moins large, que la largeur externe de la canule 4, ce qui améliore sensiblement l'étanchéité dynamique de la canule 4 avec le bouchon 20 lors de l'actionnement. De plus, l'effort de perçage est inférieur, typiquement d'environ 6N.

Dans ce mode de réalisation particulier, le bord périphérique 7 qui entoure l'ouverture 6 de la pointe de perçage n'est pas plan en surface, mais incurvé axialement, comme visible sur la figure 4. Avantageusement, ce bord périphérique ne comporte pas de profil saillant. La tête de perçage 5 est avantageusement de forme externe arrondie dans la direction radiale, comme représenté sur les figures 3 et 4. L'extrémité axiale distale 59 de ladite pointe de perçage 5 de l'invention est arrondie dans la direction axiale, comme visible sur les figures 5 et 6.

Avantageusement, ladite pointe de perçage 5 est formée par matriçage de ladite canule 4. La figure 7 illustre schématiquement un ensemble de matriçage, avec une partie inférieure 100 de déformation et une partie supérieure 200 de guidage. La partie supérieure 200 de guidage comporte un canal cylindrique 210 recevant la canule 4, et la partie inférieure 100 comporte une zone de déformation 110. Cette zone de déformation comporte avantageusement une partie d'entrée 111 de plus grand diamètre que le diamètre de la canule 4, et une partie de déformation 112 de plus petit diamètre que le diamètre de la canule 4. Lorsque la pointe de la canule est forcée dans ladite zone de déformation 110, elle va se déformer pour former la pointe représentée sur les figures 4 à 6. Plus précisément, l'extrémité de la canule 4 est dans ce cas déformée radialement pour amener l'extrémité axiale distale 59 de la pointe de perçage sur l'axe central longitudinal A. Du fait que la partie de déformation 112 a un diamètre réduit, le bord périphérique 7 de la pointe se déforme pour générer ledit rétrécissement radial 7a, afin de définir la forme de poire de l'ouverture 6, avec ladite partie distale 6a de largeur réduite. Avantageusement, ladite partie de déformation 112 est de forme arrondie, ce qui donne à ladite pointe son profil arrondi en direction de son extrémité de perçage 59, comme visible sur les figures 3 et 4.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comportant un corps (1) et une tête de distribution (2) pourvue d'un orifice de distribution (3) et déplaçable axialement par rapport audit corps (1) lors de l'actionnement, ledit corps (1) recevant un réservoir (10) contenant du produit fluide, ledit réservoir (10) comportant un tube creux (11) ayant une ouverture axiale proximale (12) fermée par un bouchon (20) adapté à coulisser de manière étanche dans ledit tube (11) lors de l'actionnement, ladite tête de distribution (2) comportant une canule creuse (4) reliée d'un côté audit orifice de distribution (3) et pourvue de l'autre côté d'une pointe de perçage (5) pourvue d'une ouverture (6) pour percer ledit bouchon (20), ladite ouverture (6) définissant un bord périphérique (7) s'étendant autour de ladite ouverture (6) et comportant une extrémité axiale distale et une extrémité axiale proximale, ladite extrémité axiale distale dudit bord périphérique (7) formant l'extrémité axiale distale (59) de la pointe de perçage (5), ladite extrémité axiale distale (59) de la pointe de perçage (5) étant disposée sur l'axe central longitudinal (A) de ladite canule (4), **caractérisé en ce que** ladite ouverture (6) comporte une partie distale (6a) de largeur réduite par rapport à la largeur de ladite canule (4), ledit bord périphérique (7) comportant un rétrécissement radial (7a) pour définir ladite partie distale (6a) de largeur réduite de ladite ouverture (6), l'extrémité axiale distale (59) de ladite pointe de perçage (5) étant de forme arrondie dans la direction axiale.

2. Dispositif selon la revendication 1, dans lequel ledit bord périphérique (7) est incurvé axialement.

3. Dispositif selon l'une quelconque des revendications précédentes, dans lequel ladite pointe de perçage (5) est formée par matriçage de ladite canule (4).

4. Dispositif selon l'une quelconque des revendications précédentes, dans lequel ladite pointe de perçage (5) a une forme externe arrondie dans la direction radiale.

5. Dispositif selon l'une quelconque des revendications précédentes, dans lequel ledit réservoir (10) est formé par un tube creux borgne (11).

6. Dispositif selon l'une quelconque des revendications précédentes, dans lequel ledit réservoir (10) contient une dose unique de produit fluide distribuée lors d'un seul actionnement du dispositif.

7. Dispositif selon l'une quelconque des revendications 1 à 5, dans lequel ledit réservoir (10) contient deux doses de produit fluide distribuées lors de deux actionnements successifs du dispositif.

## Patentansprüche

1. Ausgabevorrichtung für ein fluides Produkt, umfassend einen Körper (1) und einen Ausgabekopf (2), der mit einer Ausgabeöffnung (3) versehen und bei Betätigung in Bezug auf den Körper (1) axial verschiebbar ist, wobei der Körper (1) einen Behälter (10) aufnimmt, der fluides Produkt enthält, wobei der Behälter (10) ein Hohlrohr (11) umfasst, das eine axiale proximale Öffnung (12) aufweist, die durch einen Pfropfen (20) verschlossen ist, der dazu geeignet ist, bei Betätigung dichtend in dem Rohr (11) zu gleiten, wobei der Ausgabekopf (2) eine Hohlnadel (4) umfasst, die einerseits mit der Ausgabeöffnung (3) verbunden und andererseits mit einer Durchstechspitze (5) versehen ist, welche mit einer Öffnung (6) versehen ist, um den Pfropfen (20) zu durchstechen, wobei die Öffnung (6) einen Umfangsrand (7) definiert, der sich um die Öffnung (6) erstreckt und ein axiales distales Ende und ein axiales proximales Ende aufweist, wobei das axiale distale Ende des Umfangsrands (7) das axiale distale Ende (59) der Durchstechspitze (5) bildet, wobei das axiale distale Ende (59) der Durchstechspitze (5) auf der Mittellängsachse (A) der Hohlnadel (4) angeordnet ist, **dadurch gekennzeichnet, dass** die Öffnung (6) einen distalen Teil (6a) von geringerer Breite in Bezug auf die Breite der Hohlnadel (4) aufweist, wobei der Umfangsrand (7) eine radiale Verengung (7a) umfasst, um den distalen Teil (6a) von geringerer Breite der Öffnung (6) zu definieren, wobei das axiale distale Ende (59) der Durchstechspitze (5) in axialer Richtung von abgerundeter Form ist.

2. Vorrichtung nach Anspruch 1, wobei der Umfangsrand (7) axial gekrümmt ist.

3. Vorrichtung nach einem der vorhergehenden Ansprüche, wobei die Durchstechspitze (5) durch Gesenkformen der Hohlnadel (4) gebildet ist.

4. Vorrichtung nach einem der vorhergehenden Ansprüche, wobei die Durchstechspitze (5) eine äußere abgerundete Form in radialer Richtung hat.

5. Vorrichtung nach einem der vorhergehenden Ansprüche, wobei der Behälter (10) durch ein geschlossenes Hohlrohr (11) gebildet ist.

6. Vorrichtung nach einem der vorhergehenden Ansprüche, wobei der Behälter (10) eine einzelne Dosis fluides Produkt enthält, die bei einer einzigen Betätigung der Vorrichtung ausgegeben wird.

7. Vorrichtung nach einem der Ansprüche 1 bis 5, wobei der Behälter (10) zwei Dosen fluides Produkt enthält, die bei zwei aufeinanderfolgenden Betätigungen der Vorrichtung ausgegeben werden.

## Claims

1. A fluid dispenser device comprising a body (1), and a dispenser head (2) that is provided with a dispenser orifice (3) and that is axially movable relative to said body (1) during actuation, said body (1) receiving a reservoir (10) containing fluid, said reservoir (10) comprising a hollow tube (11) having a proximal axial opening (12) that is closed by a stopper (20) that is adapted to slide in leaktight manner in said tube (11) during actuation, said dispenser head (2) including a hollow cannula (4) that is connected at one end to said dispenser orifice (3), and that is provided at its other end with a perforating tip (5) that is provided with an opening (6) for perforating said stopper (20), said opening (6) defining a peripheral edge (7) that extends around said opening (6) and that includes a distal axial end and a proximal axial end, said distal axial end of said peripheral edge (7) forming the distal axial end (59) of the perforating tip (5), said distal axial end (59) of the perforating tip (5) being arranged on the longitudinal central axis (A) of said cannula (4), the device being **characterized in that** said opening (6) includes a distal portion (6a) of width that is small relative to the width of said cannula (4), said peripheral edge (7) including a radial narrowing (7a) for defining said distal portion (6a) of small width of said opening (6), the distal axial end (59) of said perforating tip (5) is of shape that is rounded in the axial direction.

2. A device according to claim 1, wherein said peripheral edge (7) is curved axially.

3. A device according to any preceding claim, wherein said perforating tip (5) is formed by stamping said cannula (4).

4. A device according to any preceding claim, wherein said perforating tip (5) has an outer shape that is rounded in the radial direction.

5. A device according to any preceding claim, wherein said reservoir (10) is formed by a blind hollow tube (11).

6. A device according to any preceding claim, wherein said reservoir (10) contains a single dose of fluid for dispensing during a single actuation of the device.

7. A device according to any one of claims 1 to 5, wherein said reservoir (10) contains two doses of fluid for dispensing during two successive actuations of the device.
